# EUROPEAN PATENT SPECIFICATION

(11) **EP 2 029 146 B1**
(45) Date of publication and mention of the grant of the patent: **20.12.2017**
(21) Application number: 07798313.8
(22) Date of filing: 08.06.2007
(51) Int. Cl.: A61K 31/567, A61K 31/565, A61K 9/00

(54) **METHODS TO ADMINISTER SOLID DOSAGE FORMS OF ETHINYL ESTRADIOL AND PRODRUGS THEREOF WITH IMPROVED BIOAVAILABILTY**
VERFAHREN ZUR VERABREICHUNG FESTER DOSIERUNGSFORMEN VON ETHINYL-ESTRADIOL UND PRODRUGS DAVON MIT VERBESSERTER BIOVERFÜGBARKEIT
PROCÉDÉS DESTINÉS À L'ADMINISTRATION DE FORMES POSOLOGIQUES SOLIDES D'ÉTHINYL ESTRADIOL ET DE SES PROMÉDICAMENTS PRÉSENTANT UNE BIODISPONIBILITÉ AMÉLIORÉE

(30) Priority: 08.06.2006 US 812016 P
(43) Date of publication of application: 04.03.2009
(73) Proprietor: Warner Chilcott Company, LLC, Fajardo (PR)
(72) Inventor: deVRIES, Tina, Long Valley, NJ 07853 (US); McNAMEE, Brian, Belfast, County Antrim N. Ireland BT8 6WD (GB)
(74) Representative: FRKelly
(86) International application number: PCT/US2007/070761
(87) International publication number: WO 2007/146805

(56) References cited:
- EP-A- 0 286 581
- US-A- 4 764 378
- US-A- 5 047 244
- US-B1- 6 241 529
- US-B1- 6 667 050
- ANONYMOUS: "Ovcon 35 Chewable" INTERNET ARTICLE, 15 September 2005 (2005-09-15), pages 1-3, XP002459477 Retrieved from the Internet: URL:HTTP://WWW.DRUGDIGEST.ORG>
- "Mannitol ED - Ammon H", HUNNIUS PHARMAZEUTISCHES WÖRTERBUCH, WALTER DE GRUYTER, DE, 1 January 2004 (2004-01-01), pages 1-2, XP007917997,

## Description

### BACKGROUND OF THE INVENTION

### Field of the Invention

This disclosure is directed to methods to orally administer solid dosage forms of ethinyl estradiol and prodrugs thereof with improved bioavailability. These methods avoid hepatic first pass and first pass metabolism by allowing for absorption of the ethinyl estradiol through the oral mucosa. By utilizing the methods disclosed herein, the safety profile of orally administered ethinyl estradiol is improved by reducing the dosage amount of ethinyl estradiol necessary to achieve the clinically desired bioavailable concentration in the patient. Patient compliance may also be improved as a result of reduced potential side effects.

### Related Background Art

When properly used, contraceptive compositions containing both estrogenic and progestogenic compounds are known to be highly effective in controlling ovulation and conception. The progestogenic component of the composition is primarily responsible for the contraceptive efficacy of the composition, while the estrogenic component is included to reduce undesired side effects, such as breakthrough bleeding or spotting. In fact, small amounts of estrogen help stabilize the endometrium and allow cyclic withdrawal bleeding, similar to the natural menstrual cycle.

The combination may also be used to treat symptoms of menopause. Such hormone replacement therapy is well known.

The earliest of these estrogenic/progestogenic contraceptive compositions was administered monophasically (fixed dose) and contained a relatively high level of estrogenic component. To minimize estrogen's major negative side effect on blood clotting factors, the dose of estrogen was reduced over time. However, as estrogen doses decreased, the incidences of unwanted breakthrough bleeding or spotting have generally increased.

Multiphasic oral contraceptives were introduced to artificially simulate the natural rise of progesterone over the cycle in an attempt to solve this problem. A constant goal, however, has been to reduce the estrogenic potency of such compositions without reducing contraceptive efficacy and increasing undesired side effects.

U.S. Patent No. 5,888,543 discloses various regimens where a combination of progestin and estrogen are administered in monophasic or multiphasic regimens (varied dose, e.g., biphasic or triphasic). In one embodiment, a combination of a progestin composition and an estrogen composition is administered such that the daily dosage of the second phase progestin is greater than the daily dosage of progestin in the first phase and the daily dosage of the second phase estrogen is greater than or equal to the daily dosage of estrogen in the first phase.

U.S. Patent No. 6,667,050 discloses a chewable, palatable oral contraceptive tablet, having an oral contraceptive agent, a chewable carrier suitable for human consumption, and not having a ferrocene compound. Use of the tablets in a method of human female oral contraception, and in a method of enhancing compliance with a human female oral contraception regimen is also disclosed. While it is suggested that a chewable palatable tablet could be given without liquid, there is no suggestion to choose a tablet formulation that provides for improved oral absorption or that the dosage of ethinyl estradiol in such tablet should be reduced to maintain equivalent bioavailability with the prior art tablet formulation.

U.S. Patent No. 6,974,590 discloses a pharmaceutical dosage form adapted to supply medicament to the oral cavity for buccal, sublingual or gingival absorption of the medicament, which contains an orally administrable medicament in combination with an effervescent for use in promoting absorption of the medicament in the oral cavity. The patent also discloses the use of an additional pH adjusting substance in combination with the effervescent to promote the absorption of drugs. U.S. Patent No. 6,110,486 describes a buccal polar spray or capsule that provides biologically active compounds, such as estradiol, for rapid absorption through the oral mucosa. There is no suggestion of using a solid dosage form of delivery.
U.S. Patent No. 4,764,378 discloses a buccal drug dosage form in an erodible matrix comprising low, medium and high molecular weight polyethylene glycol components. EP Patent Publication No. 0286581 discloses estrogen buccal dosage forms in a solid excipient being polyethylene glycol or a mixture thereof. An internet article retrieved from DrugDigest and entitled "Ovcon 35 Chewable" directs that, if a patient chews the Ovcon ® 35 tablet, 8 ounces of liquid should be drunk immediately afterwards so the full dose reaches the stomach and no residue is left in the mouth.

There remains a need to increase the bioavailability of hormones administered in solid dosage form thereby increasing their treatment value. When the bioavailability of hormones is increased, the effective dose required is reduced. Reduced dosing of hormones, especially estrogens, reduces unwanted side effects.

### BRIEF SUMMARY OF THE INVENTION

The present invention provides a solid dosage form for use as defined in Claims 1 to 7, an orally administered solid dosage form as defined in Claims 8 and 9, and ethinyl estradiol for use in hormone replacement therapy or for treatment of symptoms of menopause as defined in Claims 10 to 12 appended to this description. In addition, disclosed herein is a method to increase the bioavailability of solid dosage orally administered ethinyl estradiol and prodrugs thereof. Also disclosed is a method to orally administer a solid dosage form containing ethinyl estradiol without water. By reducing potential hormonal side effects and, in some cases, allowing oral administration of a solid dosage form containing ethinyl estradiol or prodrugs thereof without water, it is believed that the present methods should also improve patient compliance.

Also disclosed are methods of improving the bioavailability of ethinyl estradiol or prodrugs thereof by orally administering to a patient a solid dosage form containing ethinyl estradiol or its prodrug that releases an effective amount of the ethinyl estradiol or prodrug in the oral cavity for absorption through the oral mucosa to treat the patient for a predetermined indication. Typically, the patient is a female and the predetermined indication is oral contraception or hormone replacement therapy. Yet another embodiment of the invention is directed to an orally administered solid dosage form capable of delivering ethinyl estradiol with improved bioavailability.

The solid dosage forms are chewable tablets. Also disclosed herein are fast melt tablets (also known as orally disintegrating tablets), films, dissolving films, mucoadhesive tablets, lozenges, and chewing gum. The dosage form is a chewable tablet, and, preferably, the patient is instructed to chew the tablet prior to swallowing. Also, in a particularly preferred embodiment the patient is instructed to orally administer the dosage form, e.g., the chewable tablet, without taking water therewith.

### A BRIEF DESCRIPTION OF THE DRAWINGS

Figure 1 is a graph of mean ethinyl estradiol concentrations over time for ethinyl estradiol administered in accordance with this invention compared to prior art administration.
Figure 2 is a graph of mean norethindrone concentration over time for norethindrone acetate administered in accordance with this invention compared to prior art administration.

### DETAILED DESCRIPTION OF THE INVENTION

It surprisingly has been found that even for a solid dosage, bioavailability of ethinyl estradiol or prodrugs thereof is improved when it is absorbed buccally, sublingually, or gingivally whereby at least a portion of the administered ethinyl estradiol or prodrug avoids the digestive tract. Oral absorption allows the ethinyl estradiol or prodrugs thereof to directly enter the bloodstream avoiding hepatic first pass and first pass metabolism. It is believed that since hepatic first pass and first pass metabolism are avoided, the ethinyl estradiol or prodrugs thereof may be administered in smaller doses. In the present invention, the reduced dosing of ethinyl estradiol advantageously reduces unwanted side effects while maintaining therapeutic efficacy.

Particularly surprising has been the finding that while the bioavailability of ethinyl estradiol and its prodrugs is significantly improved when administered in accordance with the methods disclosed herein, the same inventive technique of administration has no significant impact on the bioavailability of norethindrone. This finding clearly shows that predicting the bioavailability of hormones based on the technique of administration is actually quite difficult.

As used herein, prodrugs of ethinyl estradiol are compounds having an ethinyl estradiol moiety that is cleaved or disassociated from the remaining portion of the compound upon administration and results in a therapeutically effective amount of ethinyl estradiol in the blood stream. Particularly preferred prodrugs of ethinyl estradiol are described in United States patent application No. 11/478,582, filed July 3, 2006.

The treatment methods for use of the present invention are typically undertaken once a day in a contraception or hormone replacement regimen. The effective amount of ethinyl estradiol varies depending on formulation, indication, and specific patient needs. One of skill in the art can determine the efficacy of the ethinyl estradiol in a particular treatment regimen and, thereby, determine the proper dosages based on the percent of oral absorption achieved using a particular solid dosage form and type of administration. In practicing the methods for use of the present invention, the ethinyl estradiol should be contacted with the patient's oral mucosa, whereby at least a portion, and preferably a significant portion, of the ethinyl estradiol is absorbed through the patient's oral mucosa. A suitable solid dosage form should be capable of diffusing at least a portion, and preferably a significant portion, of the ethinyl estradiol through the oral mucosa in the oral cavity. Most preferably, the solid dosage form used will result in immediate or rapid release of the ethinyl estradiol in the oral cavity.

The solid dosage form is a chewable tablet. Also, disclosed are fast melt tablets, dissolving films, mucoadhesive tablets, lozenges, and chewing gum. The choice of form will depend on the physical and chemical properties of the ethinyl estradiol or prodrug thereof as well as patient needs. In a preferred embodiment it is desirable for at least 15% of the ethinyl estradiol in the solid dosage form that is administered be absorbed through the oral mucosa, preferably at least 30%, more preferably at least 60% and most preferably at least 80%.

Preferably, the solid dosage form contains ethinyl estradiol and progestin in an amount effective for oral contraception or hormone replacement therapy in a female patient. The dosage form contains 2 µg to 15 µg of ethinyl estradiol. Preferably, when used for contraception or hormone replacement therapy, the dosage form also contains 0.3 mg to 1.5 mg of norethindrone acetate or norethindrone. Preferably, the methods for use of the present invention provide for administration of a solid dosage form once daily as part of a contraception or hormone replacement regimen.

Orally consumable films and thin strips that may be used in the method disclosed herein typically are made with a rapidly dissolvable polymer-based thin film vehicle. Consumable films and thin strips typically are administered to the oral cavity where they rapidly dissolve upon contact with saliva and provide rapid delivery of the active ingredients. Components of such films generally would include a water-soluble film-forming polymer. The consumable films may also contain other components such as a flavoring agent in and/or a surfactant. Such films are well known.

Chewing gum compositions useful in the practice of the methods disclosed herein typically would include one or more of gum base and the ethinyl estradiol. Other typical gum components would include flavoring agents and/or sweetening agent.

Lozenges are available in a variety of forms including, but not limited to, breath mints, troches, pastilles, microcapsules, and fast-dissolving solid forms including freeze dried forms (cakes, wafers, thin films, and tablets), and orally dissolvable compressed tablets. The base of a lozenge may include hard sugar candy and glycerinated gelatin. A skilled artisan can formulate a lozenge to include an active ingredient. Compressed tablet type lozenges typically include one or more fillers (e.g., compressible sugar), flavoring agents and lubricants. Of course, sugar-free compositions are also envisioned for use in the methods disclosed herein.

Another dosage form that may be preferably used in the methods disclosed herein is a fast melt tablet or orally disintegrating tablet. Such tablets are known and will be a solid dosage form, containing ethinyl estradiol or a prodrug thereof, which disintegrates rapidly, usually in a matter of seconds, when placed upon the tongue. There are many different ways to produce an orally disintegrating tablet and they include, without limitation, compressed tablets, compression molded tablets and freeze-dried (lyophilized) wafers. See e.g., Cremer, K., "Orally Disintegrating Dosage Forms", Industry summary report, Pharma Concepts GmbH R Co. (2001). Generally orally disintegrating tablets are formulated to be taken without water.

The oral dosage form for practicing the invention is a chewable tablet containing ethinyl estradiol. Most preferably, the chewable tablet will also contain the norethindrone acetate or norethindrone. The preparation of chewable tablets is shown in U.S. Patent No. 6,667,050.

However, in formulating a chewable tablet for use in the present invention one must be mindful to employ a carrier that allows for significant dissolution of the ethinyl estradiol in the oral cavity. The solid dosage form for use; orally administered solid dosage form; and ethinyl estradiol for use in hormone replacement therapy or for treatment of symptoms of menopause of the invention comprise 30% to 90% by weight of an oral dissolution enhancing carrier that is mannitol. Also disclosed herein are carriers to achieve effective dissolution including lactose, corn starch, maltodextrin, dextrose, sorbitol, xylitol, fructose, sucrose and mixtures thereof. A significant amount of dicalcium phosphate has been found not to promote the absorption required by the present invention. As also disclosed herein, the dissolution enhancing carrier will be present in an amount of about 10 to about 95 percent by weight of the composition, more preferably in an amount of about 50 to about 80 percent by weight of the solid dosage composition. As also disclosed herein, the carrier may also be a sugar alcohol such as sorbitol or xylitol. The chewable tablet may also contain other excipients generally found in such tablets, such as sweeteners, flavoring agents, disintegrants, binders and lubricants so long as the additional excipients do not substantially interfere with the oral adsorption of the ethinyl estradiol by the oral mucosa.

Another embodiment of the invention is directed to the orally administered solid dosage form that is used to practice the invention that provides improved bioavailability of ethinyl estradiol to a patient in need thereof. The solid dosage form comprises 2 µg to 15 µg of ethinyl estradiol and an oral dissolution enhancing carrier that is mannitol as described above that provides for at least 15% adsorption of the ethinyl estradiol through the oral mucosa when the solid dosage is orally administered without water. The oral dissolution enhancing carrier will be present in an amount of 30% to about 90% by weight of the composition. The solid dosage form is a chewable tablet. The preferred solid dosage form will also contain norethindrone acetate or norethindrone in an amount of 0.3 mg to 1.5 mg.

Preferably, at least about 30 percent of the ethinyl estradiol contained in the solid oral dosage form is absorbed through the oral mucosa, and more preferably at least 60 percent of the ethinyl estradiol is absorbed through the oral mucosa. The amount of ethinyl estradiol absorbed by the oral mucosa is affected by the delivery system and the residence time in the patient's mouth. Therefore, in a preferred embodiment, the patient may be instructed to administer the chewable tablet without the consumption of water or any other edible liquid while the tablet is masticated and/or immediately thereafter, e.g., less than two minutes after the tablet is completely chewed. The patient alternatively may be instructed to ingest only a small amount of water, e.g., 2 oz or less after chewing the tablet. Preferably, however, the patient would be instructed not to take water while and/or immediately after chewing the tablet.

### EXAMPLE 1 AND COMPARATIVE EXAMPLES

Tablet A is a contraceptive tablet that contains 1 mg of norethindrone acetate and 35 µg of ethinyl estradiol. Tablet B contains the same amount of norethindrone acetate and ethinyl estradiol but is formulated to be chewable. The ingredients of each tablet are set forth in Table 1 below. The bioavailability of ethinyl estradiol from masticated Chewable Tablet B administered with 2 oz of water and without water was compared with that of swallowed Tablet A that was administered with 8 oz of water.

| Table 1 | | |
|---|---|---|
| | Formulation Composition (%w/w) | |
| Ingredient | Tablet A | Tablet B |
| Norethindrone acetate | 1.43 | 1.43 |
| Ethinyl estradiol | 0.05 | 0.05 |
| Lactose | 67.53 | 12.39 |
| Mannitol | 0 | 69.53 |
| Microcrystalline cellulose | 20.00 | 10.00 |
| Sodium starch glycolate, NF | 0 | 2.00 |
| Povidone, USP | 0 | 0.06 |
| Sucralose, NF | 0 | 0.04 |
| Spearmint flavor (spray dried) | 0 | 3.0 |
| Magnesium stearate | 0 | 0.5 |
| Color | 0 | 1.0 |
| Corn Starch | 10.00 | 0 |
| Calcium stearate | 1.00 | 0 |
| TOTAL | 100 | 100 |

### Preliminary Results From Comparative Bioavailability Study

The study was a single-center, randomized, balanced, single-dose, 3-treatment, 3-period, 6-sequence crossover study conducted under medical supervision. All subjects received the following treatments in random order:
- One Tablet B chewable tablet (chewed) without water (Test 1)
- One Tablet B chewable tablet (chewed) with 2 oz of water (Test 2)
- One Tablet A tablet (swallowed whole) with 8 oz of water (Reference)

Subjects received all treatments after an overnight fast of at least 10 hours; there was a 14-day washout between administrations of the 3 treatments.

The treatments were administered after pre-dose clinical assessments and a blood sample (0 hour) was taken. The subjects remained at the clinic for the 36 hours after dosing, during which time blood samples were collected at 0.33, 0.67, 1, 1.33, 1.67, 2, 2.5, 3, 4, 6, 8, 10, 12, 16, 24, 30 and 36 hours post-treatment. Subjects then returned to the clinic for collection of further blood samples at 48 and 60 hours post-treatment. Blood samples were analyzed for plasma ethinyl estradiol (EE) and norethindrone (NE) concentrations by a validated LC/MS-MS method.

### Results and Discussion:

Twenty-seven subjects completed the study, and all 27 were evaluable for pharmacokinetic analysis.

### Preliminary Results:

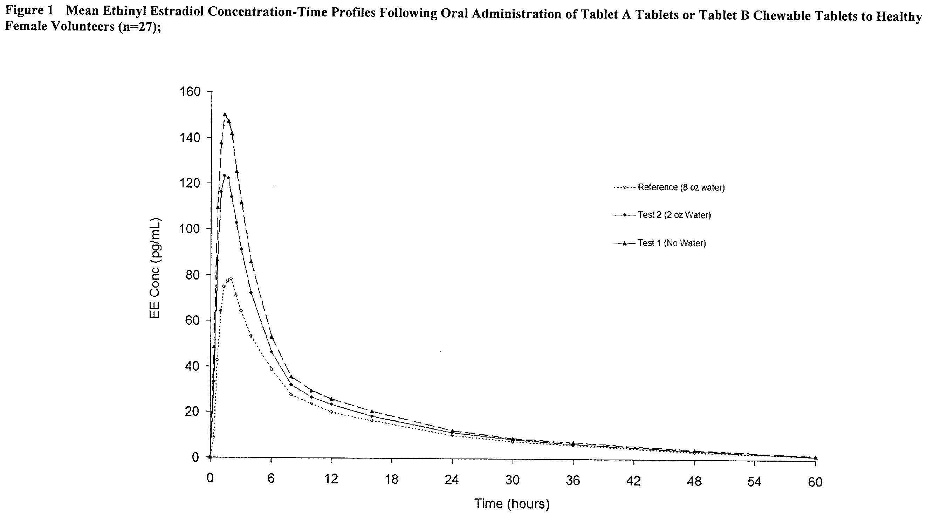

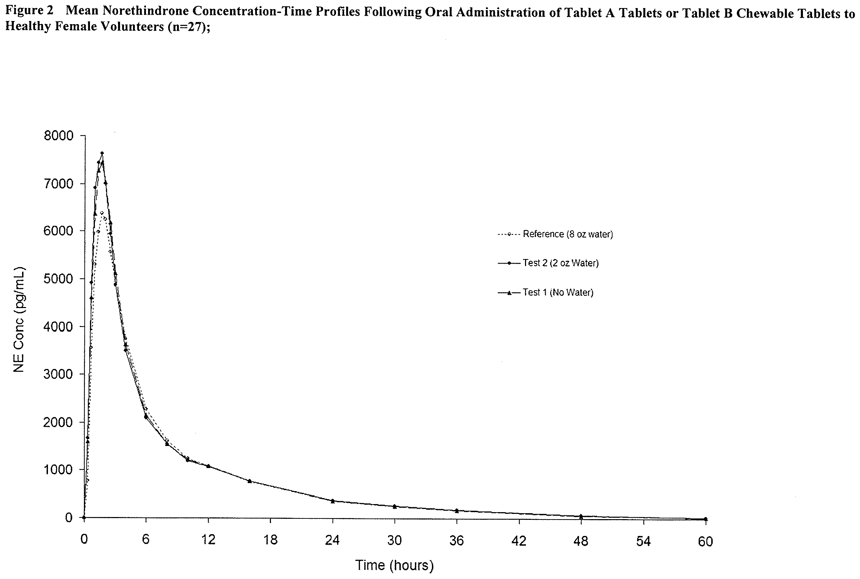

Mean plasma EE and NE concentrations are illustrated in Figures 1 and 2, respectively; a summary of the pharmacokinetic values are presented in Table 2 and the Test:Reference pharmacokinetic parameter ratios and confidence intervals are provided in Table 3.

EE and NE were rapidly absorbed; median tmax (time of Cmax) values ranged from 1.33 to 1.67 hours for both analytes following all treatments.

**Table 2 Summary of Ethinyl Estradiol and Norethindrone Pharmacokinetic Values Following Oral Administration of Tablet A Tablets or Tablet B Chewable Tablets to Healthy Female Volunteers (n=27)**

| Analyte | PK Parameter | Geometric Mean | | |
|---|---|---|---|---|
| | | Test 1 Tablet B (without water) | Test 2 Tablet B (with 2 oz water) | Reference Tablet A (with 8 oz water) |
| Ethinyl Estradiol | Cmax (pg/mL) | 154.2 | 124.8 | 78.1 |
| | AUC 0-t (pg/mL·h) | 1199.2 | 1032.7 | 805.5 |
| | AUCinf (pg/mL·h) | 1274.6 | 1096.7 | 876.0 |
| Norethindrone | Cmax (pg/mL) | 7793.0 | 7531.1 | 6646.3 |
| | AUC 0-t (pg/mL·h) | 42698.5 | 41967.5 | 39613.6 |
| | AUCinf (pg/mL·h) | 43 880.3 | 43103.4 | 40870.0 |

| | | | | |
|---|---|---|---|---|
| Cmax: Maximum plasma concentration AUC 0-t: The area under the plasma concentration versus time curve from time 0 to t, the time of last determinable concentration, as calculated by the linear trapezoidal method AUCinf: AUC 0-t plus concentration at time of last determinable concentration/ elimination rate constant. Test 1: One Tablet B chewable tablet-1/35 (chewed) without water Test 2: One Tablet B chewable tablet-1/35 (chewed) with (2 oz) of water Reference: One Tablet A tablet (swallowed whole) with (8 oz) of water | | | | |

**Table 3 Ratios and Confidence Intervals for Ethinyl Estradiol and Norethindrone Pharmacokinetic Values (n=27)**

| Analyte | | Test 1 (Tablet B Chewable without water) vs Reference | | Test 2 (Tablet B Chewable with 2 oz water) vs Reference | |
|---|---|---|---|---|---|
| | | Ratio (%) | 90% Confidence Interval | Ratio (%) | 90% Confidence Interval |
| Ethinyl Estradiol | Cmax | 197.8 | 184.2-212.3 | 160.3 | 149.3-172.1 |
| | AUC 0-t | 149.0 | 141.9-156.4 | 128.4 | 122.3-134.8 |
| | AUCinf | 145.6 | 139.0-152.5 | 125.3 | 119.7-131.3 |
| Norethindrone | Cmax | 117.1 | 108.7-126.1 | 113.0 | 104.9-121.7 |
| | AUC 0-t | 107.3 | 102.1-112.8 | 105.0 | 99.9-110.4 |
| | AUCinf | 106.9 | 101.8-112.2 | 104.5 | 99.6-109.8 |

Two treatments are considered bioequivalent if the 90% confidence intervals for Cmax and for AUC fall within 80.00% to 125.00%. Cmax is an indicator of the rate and extent of absorption; AUC is an indicator of the extent of absorption.

When administered without water, the EE Cmax value for Tablet B Chewable was 198% of that for Tablet A swallowed. The EE AUC values for the chewable tablet was 146% of that for Tablet A. The results indicate that the rate and extent of EE absorption (EE bioavailability) was significantly increased (90% confidence intervals were outside 80.00 and 125.00%) for the chewable tablet administered without water. In contrast, for norethindrone, the adsorption rate was only slightly increased and the extent of absorption was equivalent (90% confidence interval was within 80.00 to 125.00%).

When administered with 2 ounces of water (tablet pieces were washed out of the oral cavity so there was decreased opportunity for oral cavity absorption), the rate and extent of EE absorption was higher (neither Cmax nor AUC confidence intervals were within 80.00% to 125.00%), but norethindrone was bioequivalent (Cmax and AUC confidence intervals were within 80.00% to 120.00%). So the short time between chewing the tablet and taking 2 ounces of water was sufficient to significantly increase the rate and extent of EE absorption.

Using AUC 0-t the results indicate that Tablet B could be formulated with about 33% less ethinyl estradiol, but still result in a bioequivalent administration of the ethinyl estradiol administered in Tablet A when no water was administered. The results further suggest that even if 2 oz. of water were administered that the ethinyl estradiol in Tablet B could be reduced by about 22% ethinyl estradiol and remain bioequivalent with swallowed Tablet A.

Both Tablets A and B were then studied by administering the chewable Tablet B and the swallowed Tablet A with 8 ounces of water under fasting conditions. The studies were conducted twice, first with 23 subjects and then with 33 subjects. A summary of the statistical comparisons is shown in Tables 4 and 5 below.

**Table 4**

| Study 1: Summary of Statistical Comparisons (n=23) of swallowed Tablet A and chewable Tablet B | | | | |
|---|---|---|---|---|
| Parameter | Norethindrone | | Ethinyl Estradiol | |
| | Ratio | 90% Confidence Interval | Ratio | 90% Confidence Interval |
| Cmax | 101.28% | 90.81 - 112.97 | 120.29% | 112.52 - 128.59 |
| AUC 0-t | 95.93% | 88.98 - 103.43 | 116.86% | 108.68 - 125.67 |
| AUCinf | 96.09% | 89.32 - 103.38 | 115.08% | 106.83 - 123.96 |

**Table 5**

| Study 2: Summary of Statistical Comparisons (n=33) of swallowed Tablet A and chewable Tablet B | | | | |
|---|---|---|---|---|
| Parameter | Norethindrone | | Ethinyl Estradiol | |
| | Ratio | 90% Confidence Interval | Ratio | 90% Confidence Interval |
| Cmax | 117.65 | 107.53 - 128.72 | 134.82 | 127.27 - 142.81 |
| AUC 0-t | 104.90 | 98.49 - 111.71 | 114.87 | 109.86 - 120.11 |
| AUCinf | 104.93 | 98.58 - 111.69 | 114.85 | 108.83 - 119.10 |

In Study 1, the norethindrone for Tablet B chewed was bioequivalent to Tablet A (swallowed). For EE, Cmax values were higher for Tablet B chewable tablets as compared to Tablet A swallowed tablets. The extent of EE absorption (measured as AUC_{inf}) from the chewable tablet was 115% of that from the swallowed tablets.

In Study 2, for norethindrone, the extent of absorption for Tablet B chewed was equivalent to Tablet A (swallowed). The rate of norethindrone absorption was higher for Tablet B chewed than for Tablet A swallowed. For EE, rate of absorption (reflected in Cmax values) was higher for Tablet B chewable tablets as compared to Tablet A swallowed tablets. The extent of EE absorption (measured as AUC₀₋ₜ) from the chewable tablet was 115% of that from the swallowed tablets.

Although the data set forth in Tables 4 and 5 showed a tendency toward higher bioavailability of EE, the upper limit of the 90% confidence interval for AUC inf did not exceed 125.00%. This data shows the importance of allowing the dosage form to reside in the mouth for an effective amount of time. In this case, it is apparent that the 8oz of water mitigated the effect of improved dissolution in the mouth achieved with formulation B. Thus, in a highly preferred embodiment of this invention the dosage form will be administered with 2 oz of water or less and most preferably without water or any other edible liquid.

In yet another study, Tablets C and D were formulated as shown in Table 6 below. Tablet C was a chewable tablet containing 0.4 mg or norethindrone and 35 µg of ethinyl estradiol substantially similar to chewable tablets disclosed in U.S. Patent No. 6,667,050. Tablet D was a swallowed tablet containing the same amount of norethindrone and ethinyl estradiol as Tablet C.

**Table 6**

| Summary of Tablet Formulation Composition | | |
|---|---|---|
| | Formulation Composition (%w/w) | |
| Ingredient | Tablet C | Tablet D |
| Norethindrone | 0.46 | 0.46 |
| Ethinyl estradiol | 0.042 | 0.042 |
| Dibasic calcium phosphate dihydrate, USP | 45.28 | 46.32 |
| Lactose (Hydrous),NF | 45.28 | 46.32 |
| Sodium starch glycolate, NF | 4.57 | 4.57 |
| Povidone, USP | 1.71 | 1.71 |
| Sucralose, NF | 0.02 | 0 |
| Maltodextrin, NF | 0.18 | 0 |
| Spearmint flavor (spray dried) | 2.0 | 0 |
| Magnesium stearate | 0.46 | 0.46 |
| FD&C yellow #6 | 0 | 0.11 |
| TOTAL | 100 | 100 |

In a first study, both Tablets C and D were administered under fasting conditions with 8 oz of water. A summary of the statistical comparisons of these results are shown in Table 7 below.

**Table 7**

| Study 1: Summary of Statistical Comparisons of chewable Tablet C and swallowed Tablet D | | | | |
|---|---|---|---|---|
| Parameter | Norethindrone | | Ethinyl Estradiol | |
| | Ratio | 90% Confidence Interval | Ratio | 90% Confidence Interval |
| Cmax | 90.7 | 83.1 - 99.1% | 115.9 | 111.0 - 121.1% |
| AUC 0-t | 100.3 | 92.5 - 108.8% | 109.7 | 1043 - 115.4% |
| AUCinf | 102.4 | 94.4 - 111.1% | 108.2 | 103.3 - 113.3% |

In a second study, both Tablets C and D were administered under fasting conditions without water. A summary of the statistical comparisons of these results are shown in Table 8 below.

**Table 8**

| Study 2: Summary of Statistical Comparisons of chewable Tablet C and swallowed Tablet D | | | | |
|---|---|---|---|---|
| Parameter | Norethindrone | | Ethinyl Estradiol | |
| | Ratio | 90% Confidence Interval | Ratio | 90% Confidence Interval |
| Cmax | 118.3 | 110.3 - 127.0 | 122.2 | 114.8-130.2 |
| AUC 0-t | 105.2 | 101.2 - 109.3 | 114.1 | 105.0-124.0 |
| AUCinf | 104.7 | 100.7 - 108.8 | 117.4 | 110.3 - 124.9 |

The results of these studies show that when chewable Tablet C and swallowed Tablet D were given with water that they were bioequivalent. In addition, when given without water the extent of norethindrone and ethinyl estradiol absorption (AUC) was considered equivalent, although there was a trend toward higher bioavailability of ethinyl estradiol for the chewable tablet. In addition Cₘₐₓ values were slightly higher for chewable Tablet C compared to Tablet D. These results clearly show that simply administering a chewable tablet without water does necessarily guarantee improved bioavailability of ethinyl estradiol, but that it is also important that the dosage formulation provide effective dissolution of the ethinyl estradiol in the oral cavity.

Generally, the dosage formulation used in the method for use of this invention may contain at least 10% less ethinyl estradiol than prior art dosage formulations that do not provide for at least 15% oral absorption of ethinyl estradiol, and more preferably at least 30% oral absorption of ethinyl estradiol, but achieves the same ethinyl estradiol bioavailability as such prior art dosage formulation. The method for use of this invention therefore allows for the administration of a reduced amount of ethinyl estradiol to a person in need thereof compared to prior art formulations while obtaining a Cₘₐₓ and AUC that is bioequivalent to that obtained with the prior art formulation.

### EXAMPLE 2

Another chewable tablet that is useful in the method for use of the invention is formulated as follows:

**Formula Components for EE and NA Orally Disintegrating Tablet**

| **Formulation Components** | **% w/w** |
|---|---|
| Norethindrone Acetate | 1.430 |
| Ethinyl Estradiol | 0.014 |
| Lactose | 7.069 |
| Antioxidant | 0.057 |
| Mannitol | 65.830 |
| Microcrystalline Cellulose | 15.000 |
| Crosspovidone | 8.000 |
| Spearmint Flavor | 1.000 |
| Sucralose | 0.100 |
| Magnesium Stearate | 1.500 |
| Total | 100.000 |

### EXAMPLE 3

A fast melt strip that is useful in the method disclosed herein is formulated as follows:

**Formula Components for EE and NA Fast Melt Film Strip**

| **Formulation Components** | **% w/w** |
|---|---|
| Maltodextrin | 20.0 |
| Glycerol | 4.0 |
| Microcrystalline Cellulose | 6.0 |
| Alginic Acid (Sodium Salt) | 42.98 |
| Corn Starch | 25.0 |
| EE | 0.02 |
| Norethindrone Acetate | 2.00 |
| Total | 100.0 |

## Claims

1. A solid dosage form containing ethinyl estradiol for use in hormone replacement therapy or to treat symptoms of menopause,
wherein the solid dosage form is formulated to release an effective amount of the ethinyl estradiol in the oral cavity of a patient for absorption through the oral mucosa,
wherein the patient is instructed to orally administer the solid dosage form with 59.147 ml (2 ounces) of water or less, and
wherein the solid dosage form is a chewable tablet comprising
(i) 2 µg to 15 µg of ethinyl estradiol;
(ii) optionally, 0.3 mg to 1.5 mg of norethindrone acetate or norethindrone; and
(iii) 30% to 90% by weight of an oral dissolution enhancing carrier that is mannitol.

2. The solid dosage form for use according to claim 1, wherein the solid dosage form comprises 0.3 mg to 1.5 mg of norethindrone acetate or norethindrone.

3. The solid dosage form for use according to claim 1 or 2, wherein at least 15 percent of the ethinyl estradiol contained in the solid dosage form is absorbed through the oral mucosa.

4. The solid dosage form for use according to claim 1 or 2, wherein the patient is instructed to orally administer the solid dosage form without taking water therewith.

5. The solid dosage form for use according to claim 1 or 2, wherein the patient is instructed to chew the tablet prior to swallowing.

6. The solid dosage form for use according to claim 1 or 2, wherein the dosage form is administered once daily.

7. The solid dosage form for use according to claim 1 or 2, wherein the oral dissolution enhancing carrier is present in an amount of 40% to 80% by weight of the composition.

8. An orally administered solid dosage form comprising:
(i) 2 µg to 15 µg of ethinyl estradiol;
(ii) 30% to 90% by weight of an oral dissolution enhancing carrier that is mannitol;
(iii) norethindrone acetate or norethindrone in an amount of 0.3 mg to 1.5 mg, that provides for at least 15% absorption of the ethinyl estradiol through the oral mucosa when said solid dosage form is orally administered to the patient without water and
wherein the solid dosage form is a chewable tablet.

9. The solid dosage form of claim 8, wherein the oral dissolution enhancing carrier is present in an amount of 40% to 80% by weight of the composition.

10. Ethinyl estradiol for use in hormone replacement therapy or for treatment of symptoms of menopause, the use comprising a solid dosage form and 59.147 ml (2 ounces) of water or less, the use for oral administration of the solid dosage form that releases an effective amount of the ethinyl estradiol in the oral cavity for absorption through the oral mucosa, and wherein the patient is instructed to orally administer the solid dosage form,
wherein the solid dosage form is a chewable tablet comprising
(i) 2 µg to 15 µg of ethinyl estradiol;
(ii) optionally, 0.3 mg to 1.5 mg of norethindrone acetate or norethindrone; and
(iii) 30% to 90% by weight of an oral dissolution enhancing carrier that is mannitol.

11. Ethinyl estradiol for use according to claim 10, wherein the medicament comprises the solid dosage form and no water.

12. Ethinyl estradiol for use according to claim 10 or 11, wherein the oral dissolution enhancing carrier is present in an amount of 40% to 80% by weight of the composition.

## Patentansprüche

1. Feste Dosierungsform, die Ethinylestradiol enthält, für die Verwendung in der Hormonersatztherapie oder zur Behandlung von Symptomen der Menopause,
wobei die feste Dosierungsform formuliert wird, um eine wirksame Menge des Ethinylestradiols in der Mundhöhle eines Patienten für die Resorption über die Mundschleimhaut freizusetzen,
wobei der Patient zur oralen Verabreichung der festen Dosierungsform mit 59,147 ml (2 Unzen) Wasser oder weniger angewiesen wird und
wobei die feste Dosierungsform eine Kautablette ist, die Folgendes umfasst:
(i) 2 µg bis 15 µg Ethinylestradiol;
(ii) optional 0,3 mg bis 1,5 mg Norethindronacetat oder Norethindron und
(iii) 30 Gewichts-% bis 90 Gewichts-% eines die orale Auflösung verbessernden Trägers, der Mannitol ist.

2. Feste Dosierungsform für die Verwendung nach Anspruch 1, wobei die feste Dosierungsform 0,3 mg bis 1,5 mg Norethindronacetat oder Norethindron umfasst.

3. Feste Dosierungsform für die Verwendung nach Anspruch 1 oder 2, wobei mindestens 15 Prozent des Ethinylestradiols, das in der festen Dosierungsform enthalten ist, über die Mundschleimhaut resorbiert wird.

4. Feste Dosierungsform für die Verwendung nach Anspruch 1 oder 2, wobei der Patient zur oralen Verabreichung der festen Dosierungsform ohne die Einnahme von Wasser damit angewiesen wird.

5. Feste Dosierungsform für die Verwendung nach Anspruch 1 oder 2, wobei der Patient zum Kauen der Tablette vor dem Schlucken angewiesen.

6. Feste Dosierungsform für die Verwendung nach Anspruch 1 oder 2, wobei die Dosierungsform einmal täglich verabreicht wird.

7. Feste Dosierungsform für die Verwendung nach Anspruch 1 oder 2, wobei der die orale Auflösung verbessernde Träger in einer Menge von 40 Gewichts-% bis 80 Gewichts-% der Zusammensetzung vorliegt.

8. Oral verabreichte feste Dosierungsform, die Folgendes umfasst:
(i) 2 µg bis 15 µg Ethinylestradiol;
(ii) 30 Gewichts-% bis 90 Gewichts-% eines die orale Auflösung verbessernden Trägers, der Mannitol ist;
(iii) Norethindronacetat oder Norethindron in einer Menge von 0,3 mg bis 1,5 mg, die mindestens 15 % Resorption des Ethinylestradiols über die Mundschleimhaut bereitstellt, wenn die feste Dosierungsform an den Patienten ohne Wasser oral verabreicht wird, und wobei die feste Dosierungsform eine Kautablette ist.

9. Feste Dosierungsform nach Anspruch 8, wobei der die orale Auflösung verbessernde Träger in einer Menge von 40 Gewichts-% bis 80 Gewichts-% der Zusammensetzung vorliegt.

10. Ethinylestradiol für die Verwendung in der Hormonersatztherapie oder für die Behandlung von Symptomen der Menopause, wobei die Verwendung eine feste Dosierungsform und 59,147 ml (2 Unzen) Wasser oder weniger umfasst, die Verwendung für die orale Verabreichung der festen Dosierungsform, die eine wirksame Menge des Ethinylestradiols in der Mundhöhle für die Resorption über die Mundschleimhaut freisetzt, und wobei der Patient zur oralen Verabreichung der festen Dosierungsform angewiesen wird, wobei die feste Dosierungsform eine Kautablete ist, die Folgendes umfasst:
(i) 2 µg bis 15 µg Ethinylestradiol;
(ii) optional 0,3 mg bis 1,5 mg Norethindronacetat oder Norethindron und
(iii) 30 Gewichts-% bis 90 Gewichts-% eines die orale Auflösung verbessernden Trägers, der Mannitol ist.

11. Ethinylestradiol für die Verwendung nach Anspruch 10, wobei das Medikament die feste Dosierungsform und kein Wasser umfasst.

12. Ethinylestradiol für die Verwendung nach Anspruch 10 oder 11, wobei der die orale Auflösung verbessernde Träger in einer Menge von 40 Gewichts-% bis 80 Gewichts-% der Zusammensetzung vorliegt.

## Revendications

1. Forme de dosage solide contenant de l'éthinylestradiol, pour une utilisation dans une hormonothérapie substitutive ou pour traiter les symptômes de la ménopause,
où la forme de dosage solide est formulée afin de libérer une quantité efficace de l'éthinylestradiol dans la cavité orale d'un patient pour une absorption via la muqueuse orale,
où l'on demande au patient d'administrer par voie orale la forme de dosage solide avec 59,147 ml (2 onces) d'eau ou moins, et
où la forme de dosage solide est un comprimé croquable comprenant :
(i) de 2 µg à 15 µg d'éthinylestradiol ;
(ii) éventuellement, de 0,3 mg à 1,5 mg d'acétate de noréthindrone ou de noréthindrone ; et
(iii) de 30% à 90% en poids d'un véhicule d'amélioration de la dissolution orale qui est le mannitol.

2. Forme de dosage solide pour une utilisation selon la revendication 1, où la forme de dosage solide comprend de 0,3 mg à 1,5 mg d'acétate de noréthindrone ou de noréthindrone.

3. Forme de dosage solide pour une utilisation selon la revendication 1 ou 2, où au moins 15% de l'éthinylestradiol contenu dans la forme de dosage solide est absorbé via la muqueuse orale.

4. Forme de dosage solide pour une utilisation selon la revendication 1 ou 2, où l'on demande au patient d'administrer par voie orale la forme de dosage solide sans l'accompagner d'eau.

5. Forme de dosage solide pour une utilisation selon la revendication 1 ou 2, où l'on demande au patient de croquer le comprimé avant de l'avaler.

6. Forme de dosage solide pour une utilisation selon la revendication 1 ou 2, où la forme de dosage est administrée une fois par jour.

7. Forme de dosage solide pour une utilisation selon la revendication 1 ou 2, où le véhicule d'amélioration de la dissolution orale est présent selon une quantité allant de 40% à 80% en poids de la composition.

8. Forme de dosage solide administrée par voie orale, comprenant :
(i) de 2 µg à 15 µg d'éthinylestradiol ;
(ii) de 30% à 90% en poids d'un véhicule d'amélioration de la dissolution orale qui est le mannitol ;
(iii) de l'acétate de noréthindrone ou de la noréthindrone selon une quantité allant de 0,3 mg à 1,5 mg, qui fournit au moins 15% d'absorption de l'éthinylestradiol via la muqueuse orale lorsque ladite forme de dosage solide est administrée au patient par voie orale au patient sans eau, et où la forme de dosage solide est un comprimé croquable.

9. Forme de dosage solide selon la revendication 8, dans laquelle le véhicule d'amélioration de la dissolution orale est présent selon une quantité allant de 40% à 80% en poids de la composition.

10. Ethinylestradiol pour une utilisation dans une hormonothérapie substitutive ou pour traiter les symptômes de la ménopause, l'utilisation comprenant une forme de dosage solide et 59,147 ml (2 onces) d'eau ou moins, l'utilisation pour l'administration orale de la forme de dosage solide qui libère une quantité efficace de l'éthinylestradiol dans la cavité orale pour une absorption via la muqueuse orale,
et où l'on demande au patient d'administrer par voie orale la forme de dosage solide, où la forme de dosage solide est un comprimé croquable comprenant :
(i) de 2 µg à 15 µg d'éthinylestradiol ;
(ii) éventuellement, de 0,3 mg à 1,5 mg d'acétate de noréthindrone ou de noréthindrone ; et
(iii) de 30% à 90% en poids d'un véhicule d'amélioration de la dissolution orale qui est le mannitol.

11. Ethinylestradiol pour une utilisation selon la revendication 10, où le médicament comprend la forme de dosage solide et est exempte d'eau.

12. Ethinylestradiol pour une utilisation selon la revendication 10 ou 11, où le véhicule d'amélioration de la dissolution orale est présent selon une quantité allant de 40% à 80% en poids de la composition.
